# EUROPÄISCHE PATENTSCHRIFT

(11) **EP 0 340 247 B1**
(45) Veröffentlichungstag und Bekanntmachung des Hinweises auf die Patenterteilung: **28.04.1993**
(21) Anmeldenummer: 88902392.5
(22) Anmeldetag: 11.03.1988
(51) Int. Cl.: C07J 1/00, C07C 405/00, C07B 57/00

(54) **NEUES VERFAHREN ZUR ANTIPODENTRENNUNG VON BICYCLO 3.3.0]OCTAN-3,7-DION-2-CARBONSÄUREESTERN UND IHRER 7-MONOKETALE**
NEW PROCESS FOR THE ANTIPODAL SEPARATION OF BICYCLO 3.3.0]OCTANE-3,7-DION-2-CARBOXYLIC ACID ESTERS AND THEIR 7-MONOCETALS
NOUVEAU PROCEDE DE SEPARATION DES ANTIPODES D'ESTERS D'ACIDES BICYCLO 3.3.0]OCTAN-3,7-DION-2-CARBOXYLIQUES ET DE LEURS 7-MONOCETALES

(30) Priorität: 13.03.1987 DE 3708536
(43) Veröffentlichungstag der Anmeldung: 08.11.1989
(73) Patentinhaber: SCHERING AKTIENGESELLSCHAFT, 13342 Berlin (DE)
(72) Erfinder: VORBRÜGGEN, Helmut, D-1000 Berlin 27 (DE)
(86) Internationale Anmeldenummer: DE8800149
(87) Internationale Veröffentlichungsnummer: WO8807050

(56) Entgegenhaltungen:
- FR-A- 2 582 648

## Beschreibung

Die Erfindung betrifft ein Verfahren zur Herstellung von (+)-Bicyclo-[3.3.0]octan-3-on-2-carbonsauresteroidestern.

Bicyclo [3.3.0]octan-3,7-dion-2-carbonsäureester stellen wichtige Zwischenprodukte für die Herstellung von Carbacyclinderivaten und Sesquiterpenen dar und lassen sich nach einem neuen Verfahren (DE-A-3702385) leicht herstellen .

Aus der DE-A-36 18538 ist ein Verfahren zur Racematspaltung von (1SR, 5RS)-7,7-Alkylendioxy-2-alkoxycarbonyl-cis-bicyclo[3.3.0]-octan-3-on mit einem Mikroorganismus bekannt.

Es wurde nun gefunden, daß bei der Transveresterung dieser Bicyclo-[3.3.0]octan-3,7-dion-2-carbonsäureester mit natürlich konfigurierten Steroiden die eine freie 17β-Hydroxylgruppe enthalten, überraschenderweise nur die Derivate mit der in Formel I dargestellten absoluten Konfiguration auskristallisieren. während die entsprechenden diastereomeren Ester IV in Losung verbleiben und in hohen optischen Ausbeuten aus den Mutterlaugen gewonnen werden können.

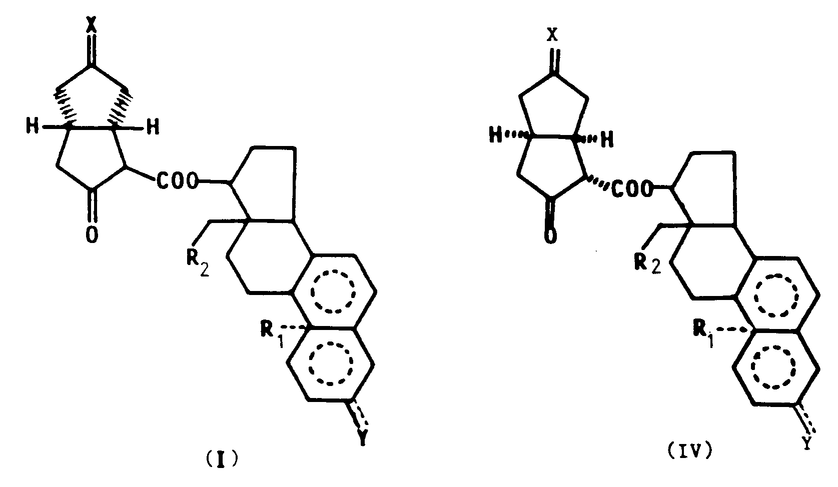

Gegenstand der Erfindung ist also Antipodentrennung der racemischen D.L-Bicyclo [3.3.0]octan-3,7-dion-carbonsaureesterderivate II durch Transveresterung mit Steroiden mit freier 17β-Hydroxylgruppe, insbesondere mit aromatischem A-Ring, der Formel III und nachfolgender Kristallisation zu Steroidestern, wobei nur die Antipoden mit der in der allgemeinen Formel I angegebenen absoluten Kofiguration fast optisch rein auskristallisiereren, während die zu I diastereomeren Steroidester IV in optischen Ausbeuten von ca 90 % aus der Mutterlauge gewonnen werden können.

Die Erfindung betrifft somit ein Verfahren zur Herstellung von (+)-Bicyclo [3.3.0] octan-3-on-2-carbonsäuresteroidestern der Formel I, 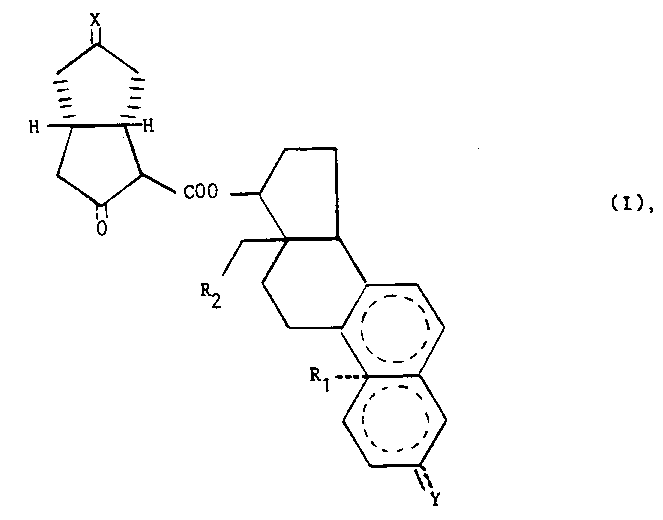
worin X Sauerstoff oder die Reste -O-(CH₂)ₙ-O- oder
-O-CH₂-C(CH₃)₂-CH₂-O-,
n 2 oder 3,
R₂ Wasserstoff oder Methyl,
Y die Reste X oder X₁,
X₁ die Reste OCH₃ oder OCOR₃,
R₃ Methyl, Ethyl, Phenyl, Benzyl oder Pivalyl und der Rest 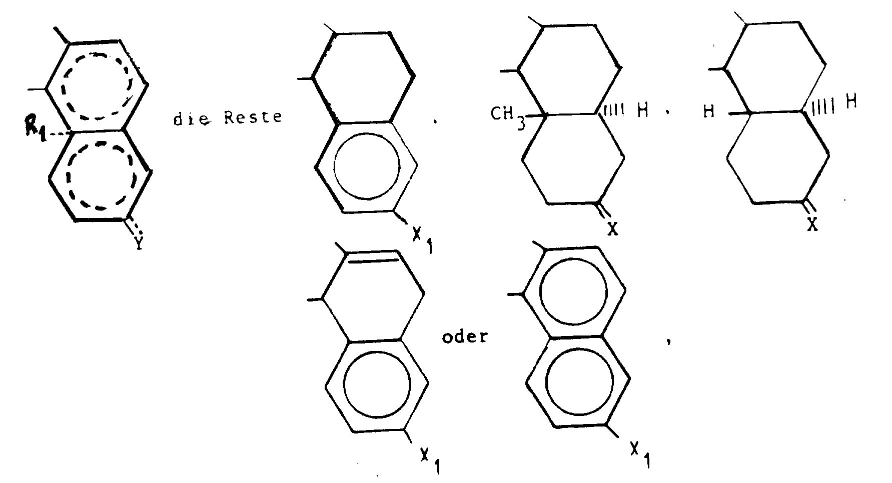
wobei X und X₁ die oben angegebenen Bedeutungen haben, bedeuten, dadurch gekennzeichnet, daß man D,L-Bicyclo [3.3.0]-octan-3-on-2-carbonsäureester der Formel II, 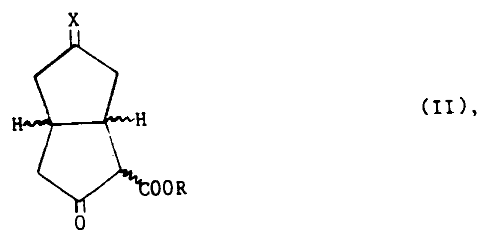
worin X die oben angegebene Bedeutung hat und R eine Alkylgruppe mit
1-4 C-Atomen darstellt, mit optisch aktiven Steroiden mit einer freien 17β-Hydroxygruppe der Formel III, 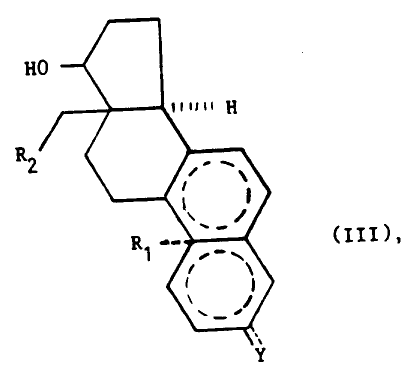
worin R₂ und der Rest 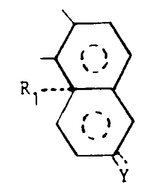
die oben angegebenen Bedeutungen haben, in Gegenwart eines nukleophilen oder sauren Katalysators in die diastereomeren Steroidester überfuhrt und die optisch reinen Steroidester der- Formel I durch Umkristallisieren in kristalliner Form erhält, während die diastereomeren Steroidester der Formel IV in Lösung bleiben und anschließend aus der Mutterlauge gewonnen werden.

Als Alkylgruppen mit 1-4 C-Atomen (für R)kommen die Reste Methyl, Ethyl, n-Propyl, Isopropyl, n-Butyl, sek.-Butyl und tert.-Butyl in Betracht.

Als Steroide der Formel III, die ein 17β-Hydroxygruppe tragen, kommen Östradiolmethylether sowie Verbindungen vom Equilin-Typ oder vom Equilenin-Typ in Frage. Als weitere Steroide seien solche genannt, in denen Y = X oder X₁ ist.

Die bevorzugten Reaktionen werden durch das folgende Schema I verdeutlicht: So laßt sich D,L-Bicyclo [3.3.0] octan-3,7-dion-2-carbonsäuremethylester VI mit Östradiolmethyläther VII in Gegenwart von 4-Dimethylaminopyridin (DMAP) oder 4-Pyrrolidinopyridin (PPY) nach der Methode von D.F. Taber et al., J.O.C. 50, 3618 (1985)bzw. durch säure-katalysierte Transveresterung in Gegenwart von Camphersulfonsäure, p-Toluol- oder Methansulfonsäure in Toluol in ca. 80 - 90 % Ausbeute transverestern. Dabei kristallisiert in 35 - 40 % Ausbeute der fast optisch reine Ester VIII aus, während der zu VIII diastereomere Ester (vergl. IV) in Lösung bleibt. Bei der selektiven Ketalisierung mit Neopentylglycol liefert VIII das Steroidesterketal X. Der optisch aktive Ketalester X läßt sich analog auch aus dem D,L-Neopentylketalmonomethylester IX durch Transveresterung mit Östradiolmethyläther VII in Gegenwart von DMAP oder PPY kristallin und praktisch optisch rein in ca. 35 - 40 % Ausbeute gewinnen, wobei das Diastereomere von X in der Mutterlauge verbleibt.

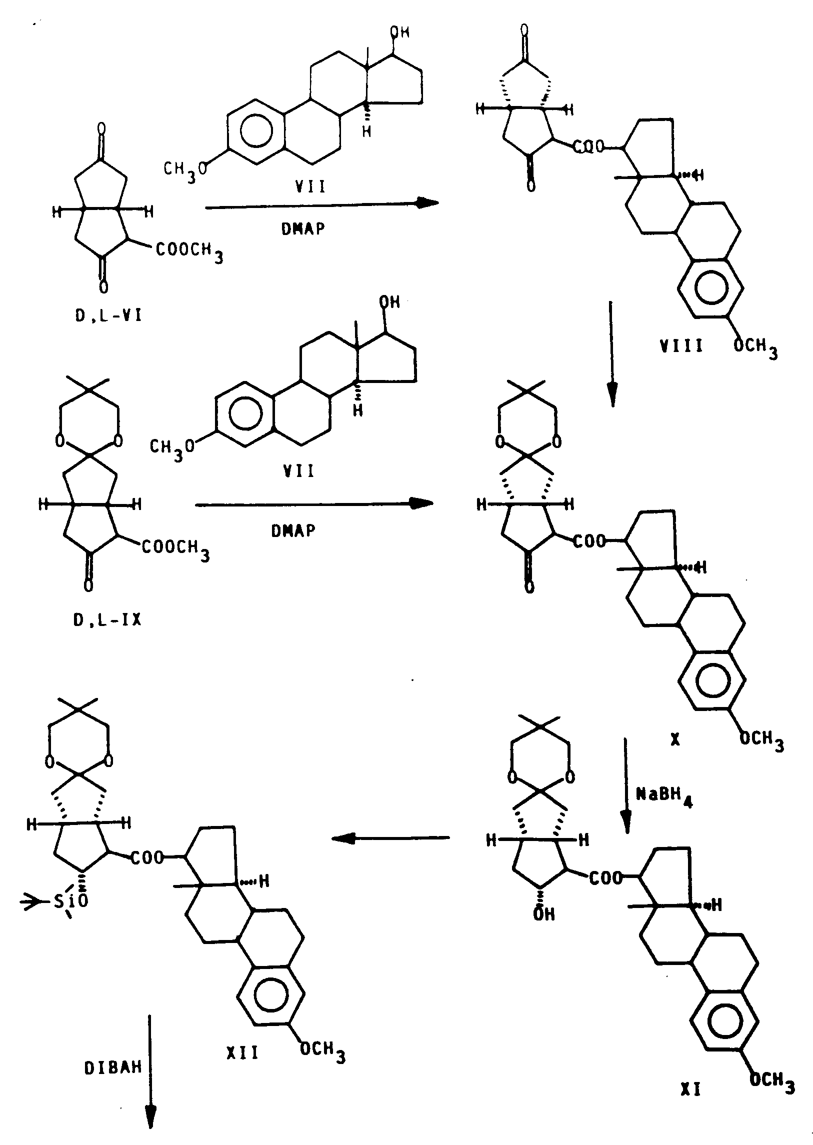

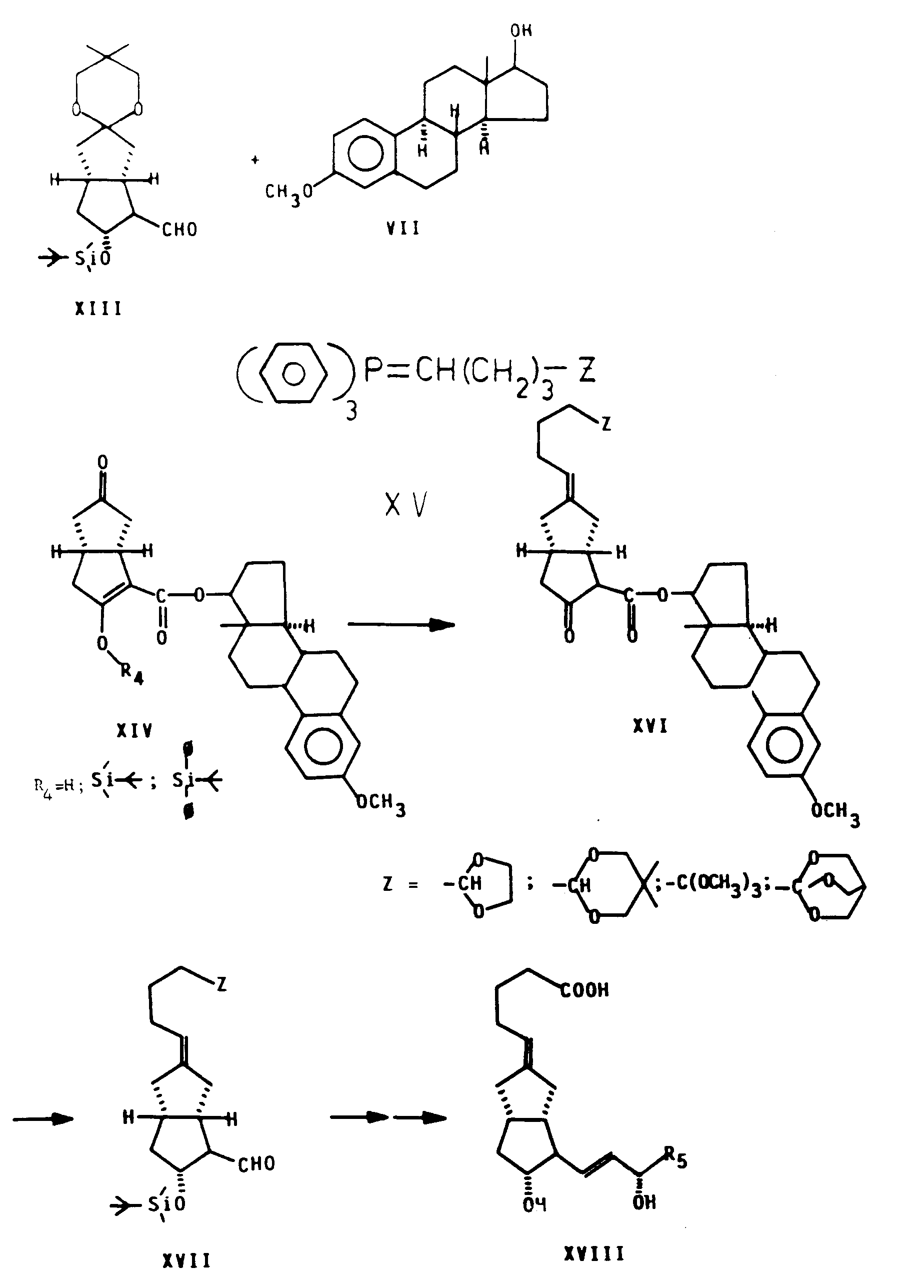

Reduktion von X mit Natriumborhydrid in Methyl-tert.-butylether-H₂O ergibt den kristallinen Alkohol XI, dessen optische Reinheit von >98 % mit Hilfe von HPLC an einer analytischen Säuee (ODS-Hypersil) mit dem System Acetonitril-Methanol-H₂O = 33 : 29 : 38 bestimmbar ist.

Reduktion des beim Abdampfen der Mutterlauge von X erhaltenen amorphen diastereomeren Steroidesters mit Natriumborhydrid in Methyl-tert.-butylether-H₂O ergibt den diastereomeren kristallinen Alkohol von XI, dessen optische Reinheit gemaß HPLC≈90 % beträgt.

Schutz der sekundären Hydroxylgruppe von XI mit tert.-ßutyl-dimethyl-silylchlorid liefert die kristalline Silylverbindung XII, die sich bei -78°Ç mit DIBAH zum Aldehyd XIII und Östadiolmethyläther VII reduzieren laßt. Nach chromatographischer Trennung Läßt sich VII wiederverwenden. Der Aldehyd XIII wird in üblicher Weise mit Wittig-Horner-Reagenzien zur Einführung der unteren Carbacyclin-Seitenkette umgesetzt.

Mit überschüssigem DIBAH erhält man in 70-90 %iger Ausbeute das ölige 2-Hydroxymethyl-3a-(dimethyl-tert.-butyl-silyloxy)-7-(2,2-dimethyl-1,3-propylendioxyl-bicyclo [3.3.0] octan.

Eine weitere Möglichkeit ist die direkte Einführung der oberen Seitenkette in XIV mit Hilfe eines 5 - 6fachen Überschusses der Wittig-Reagenzien XV zum Produkt XVI. Die nach der 5-E/Z-Trennung erhaltene reine 5-E-Verbindung XVI läßt sich analog nach Natriumborhydrid-Reduktion, Silylierung und DIBAH-Reduktion in XVII sowie nach Wittig-Horner-Reaktion, Reduktion der 15-Ketogruppe und Entfernung der Schutzgruppen in die Carbacycline XVIII überführen.

In dem Verfahren zur Herstellung von (+)-Bicyclo [3.3.0]octan-3-on-2-carbonsäuresteroidestern der Formel I werden katalytische Mengen, d.h. 0.01 - 0,5 molare Mengen, bevorzugt 0,1 Mol einer nukleophilen Base, z.B. 4-Dimethylaminopyridin, 4-Pyrrolidinopyridin, 4-Piperidino-pyridin bzw. polymerfixierte Derivate dieser Verbindungen oder N-Methylimidazol in unpolaren Lösungsmitteln, wie z.B. Dioxan, Toluol, Xylol auf 80° - 160° C, vorzugsweise auf 110 - 150° erhitzt.
Analog kann man I durch Erhitzen mit 0,01 - 0,05 molaren Mengen an Sulfonsäuren wie Camphersulfonsäure, p-Toluolsulfonsäure oder Methansulfonsäure in Toluol herstellen.

Die Erfindung umfaßt auch die (+)-Bicyclo [3.3.0]octan-3-on-2-carbonsäuresteroidester der Formel I als wertvolle Zwischenprodukte für die Herstellung von pharmakologisch wirksamen Carbacyclinen der Formel XVIII, wie z.B. das Blutdruck-senkende und Thrombozytenaggregationshemmende Iloprost bzw. als biologisch wertvolle Derivate des Östradiolmethylethers. Weiterhin dienen die Verbindungen I bzw. deren Diastereomere IV als wertvolle Zwischenprodukte für die Herstellung von Naturstoffen wie Sesquiterpenen.

### Beispiel 1:

### [(+)-3'-Methoxy-1', 3', 5' - estratrien-17'β-yl] -bicyclo [3.3.0]octan-3.7-dion-2-carboxylat

a) 78,48 g (0,4 Mol) D,L-Bicyclo [3.3.0]octan-3,7-dion-2-carbonsäuremethylester, 114,56 g (0,4 Mol) Östradiol-3-methylether und 4,89 g (0,04 Mol) 4-Dimethylaminopyridin (DMPA) wurden in 1200 ml Toluol in einem Ölbad von 130° C erhitzt und das entstehende Methanol im Gemisch mit Toluol (Destillat = 220 ml) während 4 Stunden kontinuierlich abdestilliert, wobei die Temperatur im Kolben von 105° C auf 111° C anstieg.
   Nach Abkühlen wurde die dunkle Reaktionslösung im Vakuum abgedampft und der Rückstand in 500 ml Methylenchlorid gelöst, über eine mit Methylenchlorid hergestellte Säule mit 400 g eisenfreiem Silicagel filtriert und mit 3000 ml Methylenchlorid nachgewaschen und eingeengt. Der teilweise kristalline Rückstand (ca. 200 g) wurde in 350 ml Essigester heiß gelöst und zum Abkühlen und Kristallisieren über Nacht stehengelassen. Nach Abfiltrieren und Waschen mit Essigester wurden 72,1 g (ca. 40 %) der Titelverbindung vom Schmelzpunkt 159 - 165° C erhalten. [α]ₙ = 59,5° (C = 1, CHCl₃).
b) 1.0 g (0,51 m mol) D.L- Bicyclo [3,3,0] octan-3,7-dion-2-carbonsäuremethylester VI, 1,46 g (0,51 m mol) Östradiolmethyläther VII und 3,4 mg (0,025 m Mol) wasserfreie D,L-Campher-10-sulfonsäure werden unter Rühren in 20 ml als Toluol 1 Stunde in einem Ölbad von 125° unter Abdestillieren eines Toluol-Methanolgemisches erhitzt. Das erhaltene Reaktionsgemisch wird über eine mit einem Gemisch von Toluol-Essigester (4:1) hergestellte Säule von 5 g eisenfreiem Silicagel Merck filtriert und mit 50 ml Toluol-Essigestergemisch (4:1) nachgewaschen. Das Eluat wird abgedampft und der Rückstand sofort aus 5 ml heißem Essigester umkristallisiert. In zwei Portionen K₁ = 0,57 g Schmelzpunkt 169-171° sowie K₂ = 0,258 g Schmelzpunkt 149-156° wurden insgesamt 0,828 g (36 %) VII erhalten.

### Beispiel 2:

### [(+)-3'-Methoxy-1', 3', 5'-estratrien-17β-yl]-7-(2,2-dimethyl-1,3-propylendioxy)-bicyclo [3.3.0]octan-3-on-2-carboxylat

a) 50 g (0:177 Mol) (D,L)-7-(2,2-Dimethyl-1,3-propylendioxy)-bicyclo [3.3.0]octan-3-on-carbonsäuremethylester, 52,98 g (0,186 Mol) Östradiolmethylether und 2,16 g (0,0177 Mol) 4-Dimethylaminopyridin (DMAP) wurden in 1000 ml Xylol 2 h im Ölbad auf 160° C Badtemperatur erhitzt und gleichzeitig ca. 440 ml Xylol-Methanolgemisch abdestilliert. Die restliche Lösungsmittelmenge destillierte man im Vakuum ab und filtrierte die 110 g bräunlichen Rückstand nach Lösen in 1000 ml Methylenchlorid über eine mit Methylenchlorid bereitete Säule von 600 g eisenfreiem Silicagel (Merck) und wusch mit 2500 ml Methylenchlorid nach. Nach Abdampfen des Filtrats wurden die 90 g des öligen Rückstandes in 1100 ml kochendem Hexan gelöst, filtriert und zum Abkühlen stehengelassen. Die Kristalle (32,8 g) vom Schmelzpunkt 100 - 110° C wurden abfiltriert und das Filtrat auf 500 ml eingeengt, wobei beim Abkühlen weitere 8,49 g vom Schmelzpunkt 95 - 99° C anfielen. Insgesamt wurden also 41,7 g (43,5 %) erhalten. Umkristallisation aus Hexan ergab 32,8 g (34 %) Kristalle vom Schmelzpunkt 116 - 120° C.
b) 50 g (110,97 mmol) der nach Beispiel 1 erhaltenen Verbindung, 12,14 g (116,52 mmol) 2,2-Dimethylpropandiol und 0,734 g (5,55 mmol) D,L-Camphersulfonsäure sowie 100 g wasserfreies bei 200° C im Vakuum getrocknetes Magnesiumsulfat wurden in 250 ml trockenem, alkoholfreiem Methylenchlorid 18 Stunden bei 24° C gerührt, filtriert und mit Methylenchlorid gewaschen. Das nach Extraktion des Filtrats mit eiskalter Natriumkarbonatlösung, Trocknen (Na₂SO₄) und Abdampfen erhaltene gelbe Öl (68,8 g) wurde mit Hexan-Äther 8 : 2 an einer Säule von 340 g eisenfreiem Kieseigel chromatographiert. Die ersten 4 l Eluat ergaben nach Abdampfen 43 g (72 %) der rohen kristallinen Titelverbindung, die aus 300 ml Hexan die erste Portion vom 27,38 g, Schmelzpunkt 131-133° C, [α]_{D} = + 83° (C = 1, CHCl₃) und bei Einengen der Mutterlauge eine zweite Portion von 3,5 g, Schmelzpunkt 128-130° C, lieferte. Die Gesamtausbeute betrug 30,88 g = 51,8 %. Durch Elution der Säule mit 1,5 l Methanol und Abdampfen des Eluats konnten 17,3 g rohes Ausgangsmaterial zurückgewonnen werden.

### Beispiel 3:

### [(+)-3'-Methoxy-estratrien-17β-yl] -7-(2,2-dimethyl-1,3-propylendioxy)-bicyclo [3.3.0]octan-3α-ol-2-carboxylat

27.1 g (50,5 mmol) der nach Beispiel 2 erhaltenen Verbindung wurden in 1400 ml frisch destillierten Methyl-tert.-butylether und 5,5 ml H₂O suspendiert und unter Rühren 4,28 g (126,25 mmol) Natriumborhydrid zugegeben. Nach 72 h Rühren wurden 500 ml H₂O zugesetzt und die wässrige Phase mehrfach mit Methylenchlorid extrahiert. Nach Trocknen (Na₂SO₄) und Abdampfen wurden 28,6 g Rohprodukt erhalten, das nach Lösen in 150 ml Methanol bei 0° C kristallisierte. Nach Filtration und Waschen mit eiskaltem Methanol erhielt man 13,16 g farblose Kristalle vom Schmelzpunkt 120 - 125° C. Konzentration der Mutterlauge auf 60 ml und 40 ml lieferten zwei weitere Portionen von 3,71 g, F. 99 - 105° C, und 0,76 g, F. 95 - 106° C, d.h. insgesamt 17,57 g (64,6 %). Umkristallisation aus Methanol ergab die reine Titelverbindung vom Schmelzpunkt 139 - 140° C,[α]_{D} = + 55,2° (C = 1, CHCl₃).

### Beispiel 4:

### [(+)-3'-Methoxyestratrien-17'β-yl] -3α₋(dimethyl-tert.-butyl-silyloxy)-7-(2,2-dimethyl-1,3-propylendioxy)-bicylo [3.3.0]octan-2-carboxylat

1 g (1,86 mmol) der nach Beispiel 3 erhaltenen Verbindung, 0,47 g (3,06 mmol) Dimethyl-tert.-butyl-silylchlorid, 0,25 g (2,04 mmol) 4 -Dimethylaminopyridin (DMAP) sowie 0,42 ml (3,06 mmol) Triethylamin wurden 18 h bei 24° C gerührt, mit Methylenchlorid verdünnt und die Lösung über 3 g Silicagel filtriert. Nach Abdampfen des Filtrats wurde der kristalline Rückstand (1,7 g) mit 20 ml Hexan extrahiert und die Lösung nach Filtration abgedampft. Den Rückstand (1,48 g) chromatographierte man an 15 g Silicagel mit Hexan-Essigester 9 : 1. Die ersten 200 - 250 ml Eluat wurden abgedampft und der Rückstand (1,17 g) aus 10 ml Isopropanol kristallisiert, wobei 0,72 g an farblosen Kristallen vom Schmelzpunkt 94 - 97° C,[α]_{D} +27,2° (C = 1, CHCl₃) erhalten wurden. Konzentration der Mutterlauge auf 3 ml lieferte weitere 0,15 g farblose Kristalle vom Schmelzpunkt 95 - 98° C. Die Gesamtausbeute an Titelverbindung betrug 0,87 g (71,9 %)

### Beispiel 5 :

### 2-Formyl-3α-(dimethyl-tert.-butylsilyloxy)-7-(2,2-dimethyl-1,3-propylendioxy)-bicyclo [3.3.0] octan

Zu einer Lösung von 1,6 ml (1,915 mmol) Diisobutylaluminiumhydrid (DIBAH) in Toluol wurde bei -78° C innerhalb von 15 min. eine Lösung von 0,5 g (0,766 mmol) der nach Beispiel 4 erhaltenen Verbindung in 6,5 ml abs. Methylenchlorid zugetropft und 1 h bei - 78° C belassen. Nach weiterer Zugabe von 1,6 ml (1,915 mmol) DIBAH-Lösung in Toluol wurde eine weitere Stunde bei - 78° C gerührt, dann 1,33 ml Isopropanol vorsichtig zugetropft und schließlich 1,33 ml H₂O und 3,5 ml Methylenchlorid zugegeben. Nach einer weiteren Stunde bei 78° C Ließ man auf 24° C erwärmen und filtrierte die Salze ab, die sorgfältig mit Methylenchlorid nachgewaschen wurden. Nach Trocknen (Na₂SO₄) und Einengen chromatographierte man den Rückstand (0,56 g) an einer Säule von 17 g Silicagel mit Hexan-Essigester 95 : 5, wobei 90 g (32 %) der reinen farblosen öligen Titelverbindung isoliert wurden.

Elution der SiO₂-Säule mit Methanol, Extraktion mit Hexan ergab nach Kristallisation 170 mg reinen kristallinen Östradiolmethylether.

Mit überschüssigen DIBAH erhält man in 70 - 90 % Ausbeute an Stelle der Titelverbindung das ölige 2-Hydroxymethyl-3α-(dimethyl-tert.-butylsilyloxy)-7-(2,2-dimethyl-1,3-propylendioxy)-bicyclo [3.3.0]-octan.

## Patentansprüche

1. Verfahren zur Herstellung von (+)-Bicyclo [3.3.0]octan-3-on-2-carbonsauresteroidestern der Formel I und seiner diastereomeren Steroidester der Formel IV. 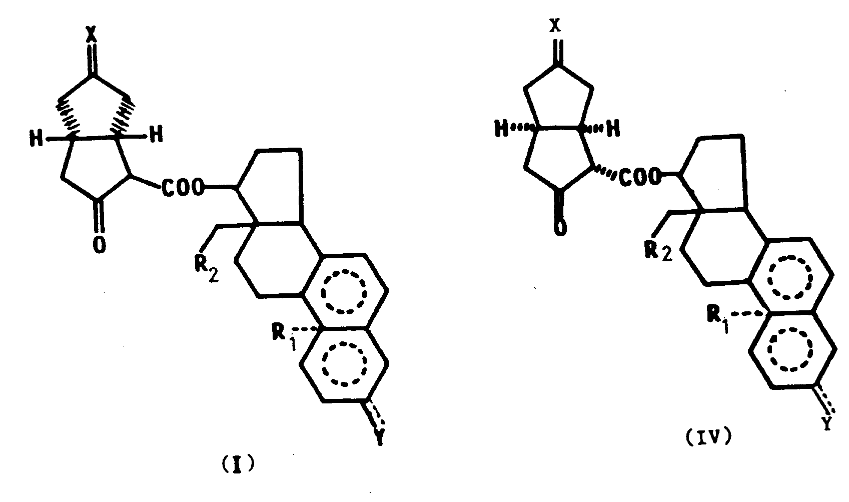 worin
X Sauerstoff oder die Reste -O-(CH₂)ₙ-O- oder
-O-CH₂-C(CH₃)₂-CH₂-O-,
n 2 oder 3.
R₂ Wasserstoff oder Methyl.
Y die Reste X oder X₁,
X₁ die Reste OCH₃ oder OCOR₃,
R₃ Methyl, Ethyl, Phenyl, Benzyl oder Pivalyl und der Rest 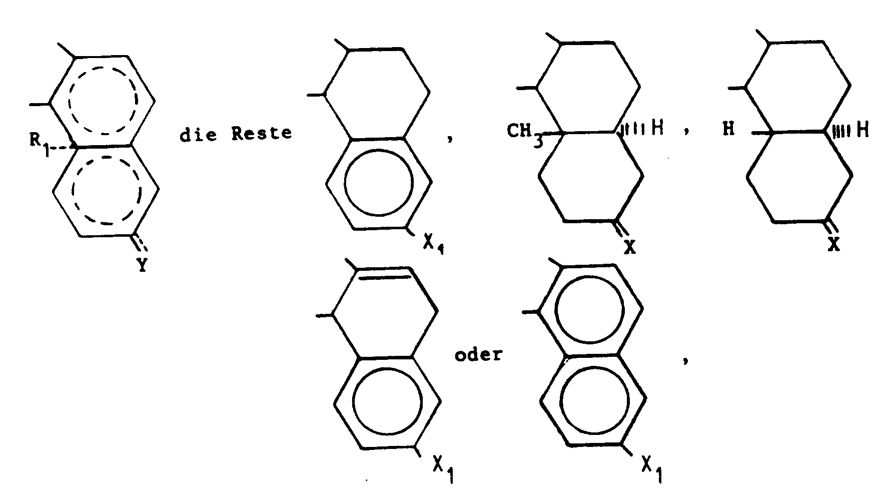 wobei X und X₁ die oben angegebenen Bedeutungen haben, bedeuten, dadurch gekennzeichnet, daß man D,L-Bicyclo-[3.3.0] -octan-3-on-2-carbonsaureester der der Formel II, 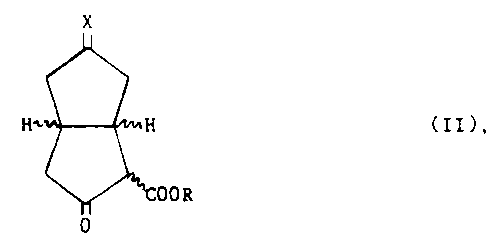 worin X die oben angegebene Bedeutung hat und R eine Alkylgruppe mit
1-4 C-Atomen darstellt, mit optisch aktiven Steroiden mit einer freien 17'β-Hydroxygruppe der Formel III, 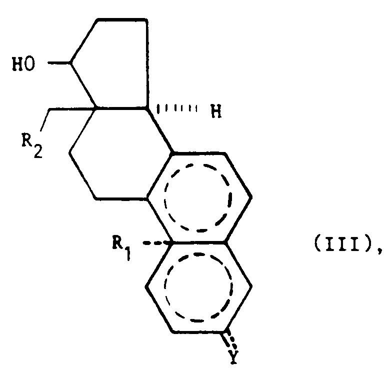 worin R₂ und der Rest 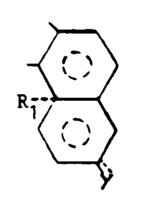 die oben angegebenen Bedeutungen haben, in Gegenwart eines nukleophilen oder sauren Katalysators in die diastereomeren Steroidester überführt und die optisch reinen Steroidester der Formel I durch Umkristallisieren in kristalliner Form erhält, während die diastereomeren Steroidester der Formel IV in Lösung bleiben und anschließend aus der Mutterlauge gewonnen werden.

2. Verfahren nach Anspruch 1, dadurch gekennzeichnet, daß man als optisch aktives Steroid der Formel III Östradiolmethylether verwendet.

3. Verfahren nach Anspruch 1, dadurch gekennzeichnet, daß man als nukleophilen Katalysator 4-Dimethylaminopyridin, 4-Pyrrolidinopyridin, 4-Piperidinopyridin oder die polymerfixierten Derivate dieser Basen oder N-Methyl-imidazol verwendet oder mit den sauren Katalysatoren Camphersulfonsäure, p-Toluol- oder Methansulfonsäure in unpolaren Lösungsmitteln auf 80 - 160° C erhitzt.

4. (+)-Bicyclo [3.3.0.] octan-3-on-2-carbonsäuresteroidester der Formel I. 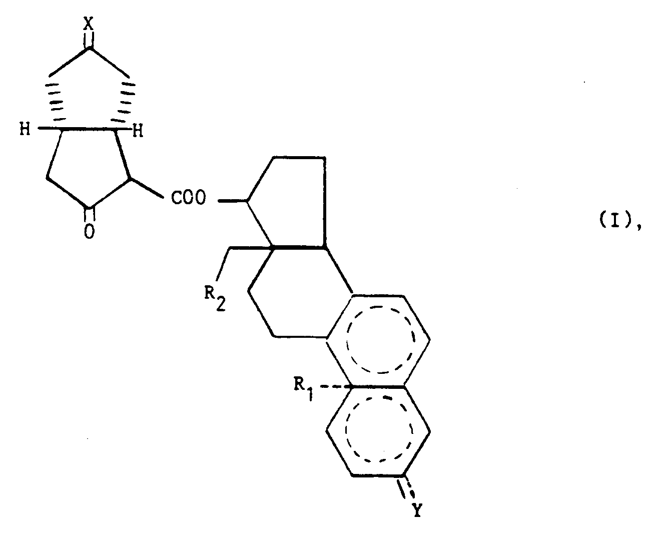 worin
X Sauerstoff oder die Reste -O-(CH₂)ₙ-O- oder
-O-CH₂-C(CH₃)₂-CH₂-O-,
n 2 oder 3.
R₂ Wasserstoff oder Methyl,
Y die Reste X oder X₁,
X₁ die Reste OCH₃ oder OCOR₃,
R₃ Methyl, Ethyl, Phenyl, Benzyl oder Pivalyl und der Rest 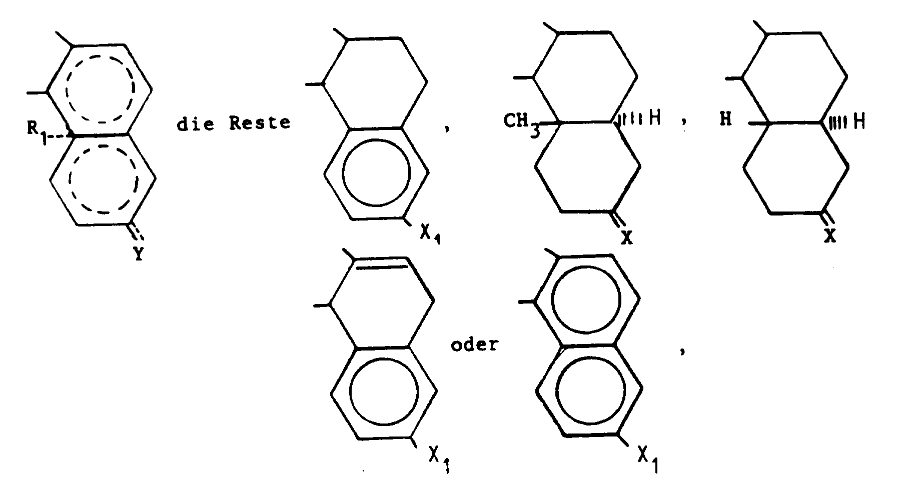 wobei X und X₁ die oben angegebenen Bedeutungen haben, bedeuten,

5. [+]-3'-Methoxy-1', 3' , 5'-estratrien-17'β₋yl] -bicyclo [3.3.0]-octan-3,7-dion-2-carboxylat.

6. [(+)-3'-Methoxy-1',3',5'-estratrien-17'β-yl] -7-(2,2-dimethyl-1 ,3-propylendioxy)-bicyclo [3.3.0] octan-3-on-2-carboxylat.

7. Bicyclo [3.3.0] octan-3-on-2-carbonsäuresteroidester der Formel IV, 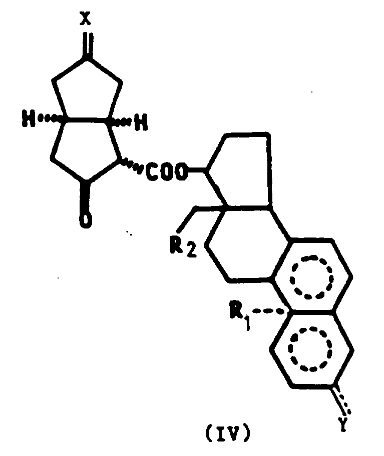 worin
X Sauerstoff oder die Reste -O-(CH₂)ₙ-O- oder
-O-CH₂-C(CH₃)₂-CH₂-O-,
n 2 oder 3,
R₂ Wasserstoff oder Methyl,
Y die Reste X oder X₁,
X₁ die Reste OCH₃ oder OCOR₃,
R₃ Methyl, Ethyl, Phenyl, Benzyl oder Pivalyl und der Rest 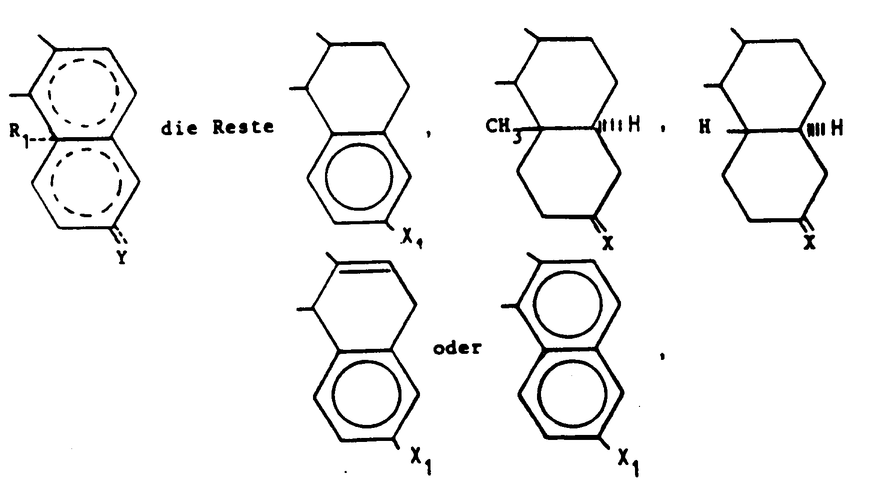 wobei X und X₁ die oben angegebenen Bedeutungen haben, bedeuten.

## Claims

1. A process for the preparation of (+)-bicyclo[3.3.0]-octan-3-one-2-carboxylic acid steroid esters of formula I and their diastereoisomeric steroid esters of formula IV 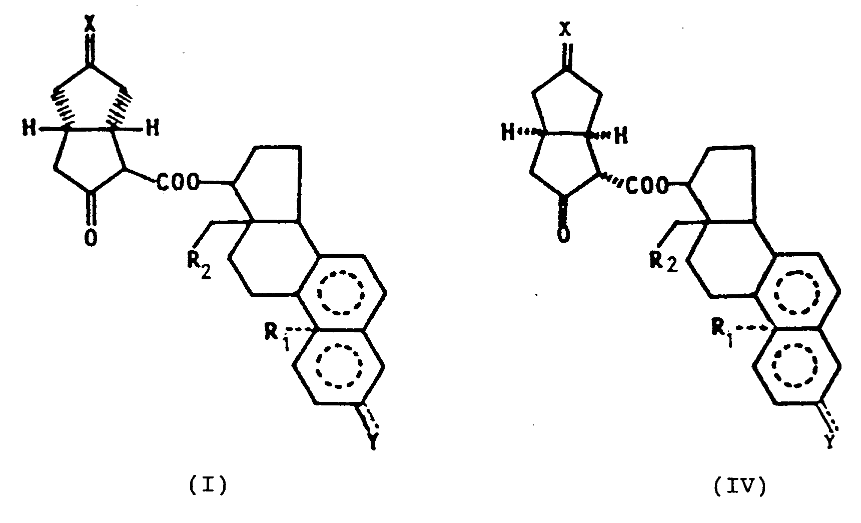 wherein
X represents oxygen or an -O-(CH₂)n-O- or
-O-CH₂-C(CH₃)₂-CH₂-O- radical,
n represents 2 or 3,
R₂ represents hydrogen or methyl,
Y represents the radical X or X₁
X₁ represents an OCH₃ or OCOR₃ radical,
R₃ represents methyl, ethyl, phenyl, benzyl or pivalyl, and the radical 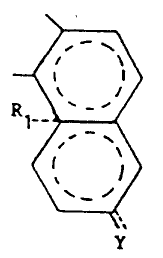 represents the radical 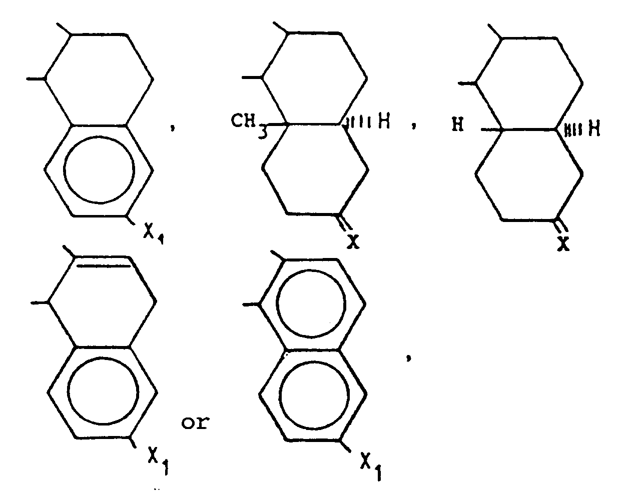 wherein X and X₁ have the meanings given above, characterised in that D,L-bicyclo[3.3.0]octan-3-one-2-carboxylic acid esters of formula II 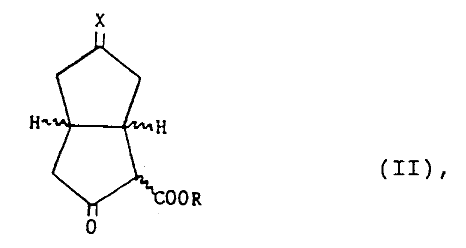 wherein X has the meaning given above and R represents an alkyl group having from 1 to 4 carbon atoms, is converted into the diastereoisomeric steroid esters using optically active steroids of formula III having a free 17'β-hydroxy group 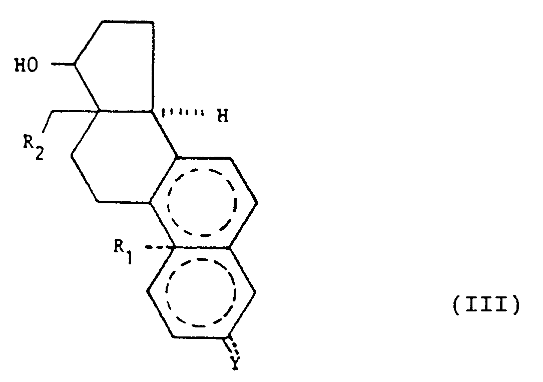 wherein R₂ and the radical 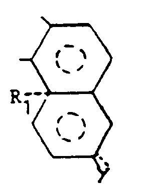 have the meanings given above, in the presence of a nucleophilic or acidic catalyst, and the optically pure steroid esters of formula I are obtained in crystalline form by means of recrystallisation whilst the diastereoisomeric steroid esters of formula IV remain in solution and are subsequently recovered from the mother liquor.

2. Process according to claim 1, characterised in that oestradiol methyl ether is used as optically active steroid of formula III.

3. Process according to claim 1, characterized in that there is used as nucleophilic catalyst 4-dimethylaminopyridine, 4-pyrrolidinopyridine, 4-piperidinopyridine or the polymer-fixed derivatives of those bases or N-methylimidazole, or heating to 80-160°C is carried out with the acidic catalysts camphorsulphonic acid, p-toluenesulphonic acid or methanesulphonic acid in non-polar solvents.

4. (+)-Bicyclo[3.3.0]octan-3-one-2-carboxylic acid steroid esters of formula I 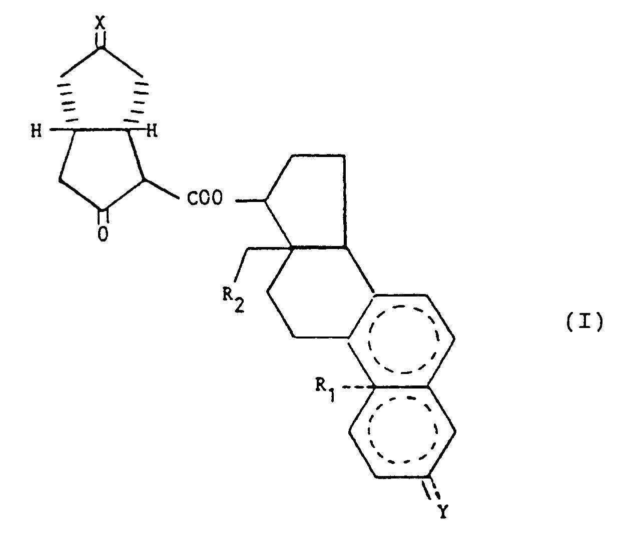 wherein
X represents oxygen or an -O-(CH₂)ₙ-O- or
-O-CH₂-C(CH₃)₂-CH₂-O- radical,
n represents 2 or 3,
R₂ represents hydrogen or methyl,
Y represents the radical X or X₁
X₁ represents an OCH₃ or OCOR₃ radical,
R₃ represents methyl, ethyl, phenyl, benzyl or pivalyl, and the radical 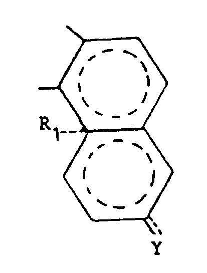 represents the radical 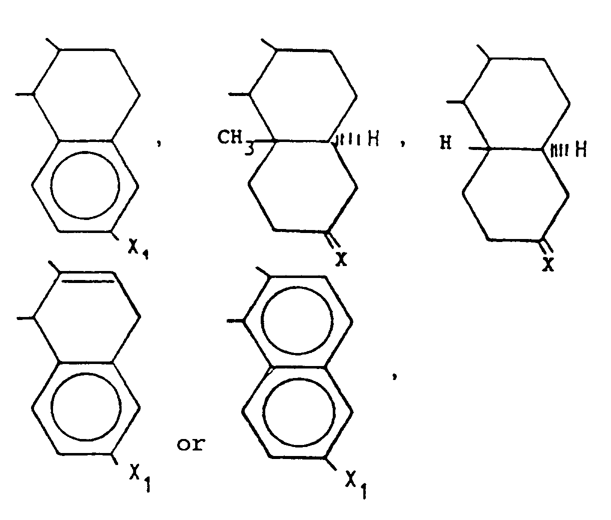 wherein X and X₁ have the meanings given above.

5. [(+)-3'-Methoxy-1',3',5'-oestratrien-17'β₋yl]-bicyclo-[3.3.0]octane-3,7-dione-2-carboxylate.

6. [(+)-3'-Methoxy-1',3',5'-oestratrien-17'β-yl]-7-(2,2-dimethyl-1,3-propylenedioxy)-bicyclo[3.3.0 ]octan-3-one-2-carboxylate.

7. Bicyclo[3.3.0]octan-3-one-2-carboxylic acid steroid esters of formula IV 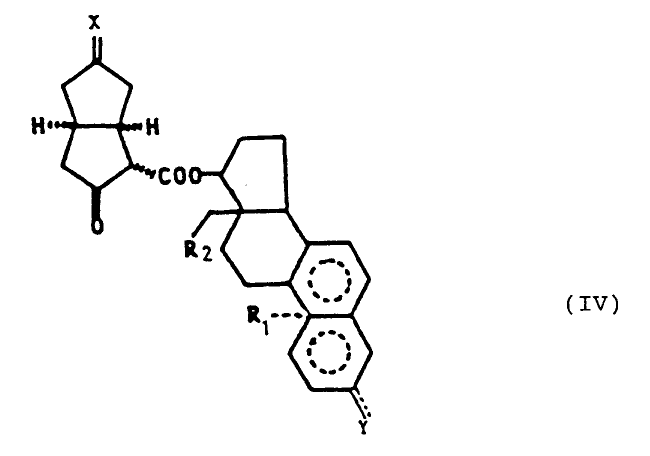 wherein
X represents oxygen or an -O-(CH₂)n-O- or
-O-CH₂-C(CH₃)₂-CH₂-O- radical,
n represents 2 or 3,
R₂ represents hydrogen or methyl,
Y represents the radical X or X₁
X₁ represents an OCH₃ or OCOR₃ radical,
R₃ represents methyl, ethyl, phenyl, benzyl or pivalyl, and the radical 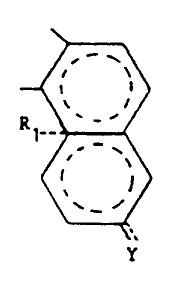 represents the radical 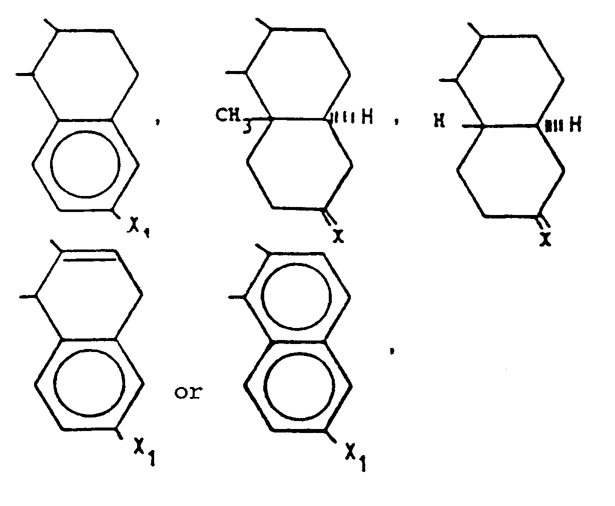 wherein X and X₁ have the meanings given above.

## Revendications

1. Procédé de préparation d'esters stéroïdes d'acides (+)-bicyclo-[3.3.0]octane-3-one-2-carboxyliques de formule I ci-dessous et de leurs diastéréomères de formule IV 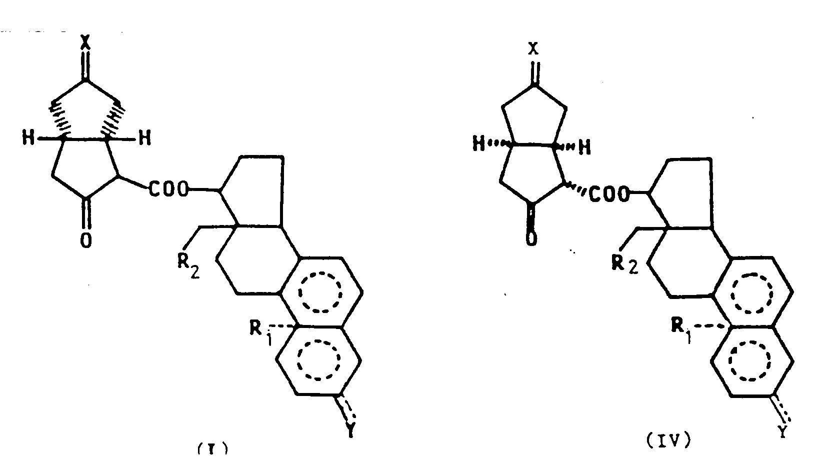 formules dans lesquelles
X désigne l'oxygène ou un radical -O-(CH₂)ₙ-O- ou -O-CH₂-
C(CH₃)₂-CH₂-O-,
n étant le nombre 2 ou 3,
R₂ l'hydrogène ou le groupe méthyle,
Y a la signification de X ou X₁,
X₁ étant un radical OCH₃ ou OCOR₃,
R₃ représentant le groupe méthyle, éthyle, phényle, benzyle ou pivalyle, et le radical 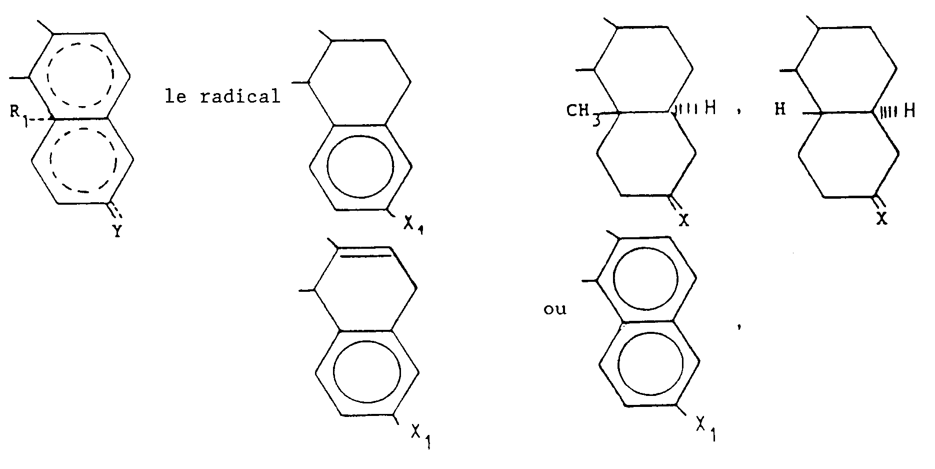 X et X₁ ayant les significations ci-dessus, procédé caractérisé en ce que l'on transforme en les esters stéroïdes diastéréomères correspondants des esters d'acides D,L-bicyclo-[3.3.0]octane-3-one-2-. carboxyliques de formule II 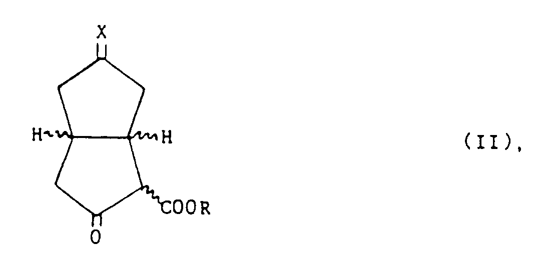 X ayant la signification précédente et R désignant un alkyle en C₁₋₄, avec des stéroïdes optiquement actifs ayant un groupe hydroxylique libre à la position 17β, de formule III 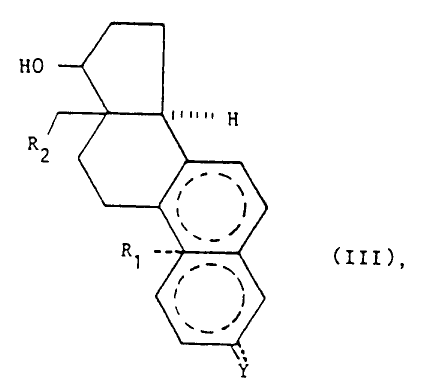 R₂ et le radical 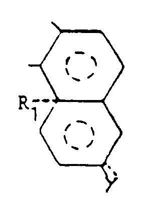 ayant les significations précédemment données, en présence d'un catalyseur nucléophile ou acide, et par recristallisation on obtient à l'état cristallisé les esters stéroïdes de formule I optiquement purs, alors que les esters stéroïdes diastéréomères de formule IV restent en solution et sont ensuite récupérés de la liqueur mère.

2. Procédé selon la revendication 1, caractérisé en ce que le stéroïde optiquement actif de formule III employé est l'éther méthylique de l'oestradiol.

3. Procédé selon la reveridication 1, caractérisé en ce qu'on utilise comme catalyseur nucléophile la 4-diméthylaminopyridine, la 4-pyrrolidonopyridine, la 4-pipéridinopyridine ou les dérivés de ces bases immobilisés sur des matières polymères ou encore le N-méthylimidazole , ou bien on chauffe avec des catalyseurs acides, acide camphosulfonique, p-toluonèsulfonique ou méthanesulfonique, dans des solvants non polaires, entre 80 et 160°C.

4. Esters stéroïdes d'acides (+)-bicyclo-[3.3.0]octane-3-one-2-carboxyliques de formule I 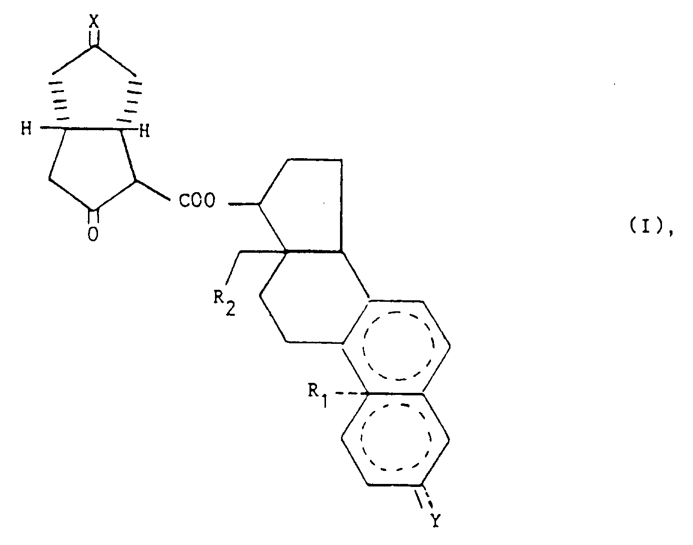 dans laquelle
X désigne l'oxygène ou le radical -O-(CH₂)ₙ-O- ou -O-CH₂-
C(CH₃)₂-CH₂-O-,
n étant le nombre 2 ou 3,
R₂ l'hydrogène ou le groupe méthyle,
Y le radical X ou X₁,
X₁ étant un radical OCH₃ ou OCOR₃ ,
R₃ représentant le groupe méthyle, éthyle, phényle, benzyle ou pivalyle, et le radical 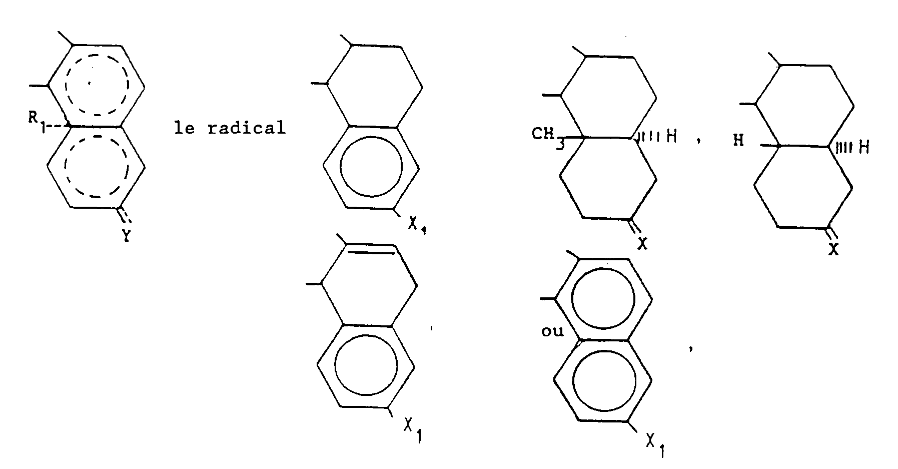 X et X₁ ayant les significations ci-dessus.

5. [(+)-3-méthoxy-1',3',5'-estratriène-17'β-yl]-bicyclo-(3.3.0]-octane-3,7-dione-2-carboxylate.

6. [(+)-3-méthoxy-1',3',5'-estracriène-17β'-yl]-7-(2,2-diméthyl-1,3-propylène-dioxy)-bicyclo[3.3.0]octane-3-one-2-carboxylate.

7. Esters stéroïdes d'acides bicyclo-[3.3.0]octane-3-one-2-carboxyliques de formule IV 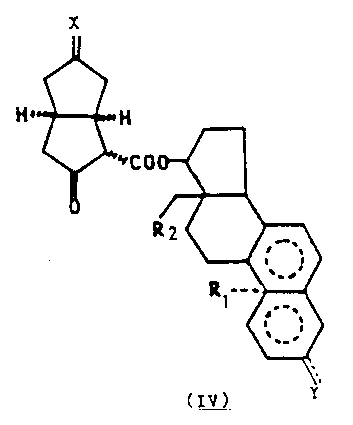 dans laquelle
X désigne l'oxygène ou le radical -O-(CH₂)ₙ-O- ou -O-CH₂-
C(CH₃)₂-CH₂-O-,
n étant le nombre 2 ou 3,
R₂ l'hydrogène ou le groupe méthyle,
Y le radical X ou X₁,
X₁ étant un radical OCH₃ ou OCOR₃,
R₃ représentant le groupe méthyle, éthyle, phényle, benzyle ou pivalyle, et le radical 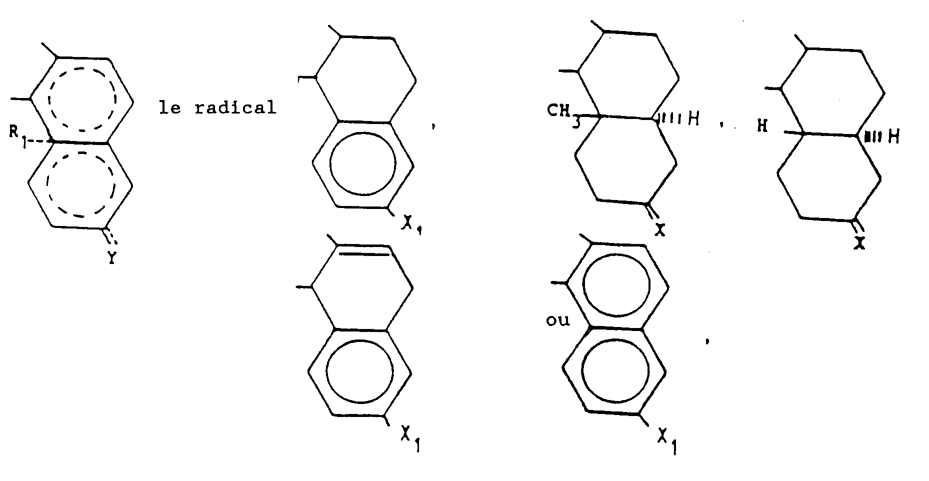 X et X₁ ayant les significations ci-dessus.
